# EUROPEAN PATENT SPECIFICATION

(11) **EP 3 423 362 B1**
(45) Date of publication and mention of the grant of the patent: **04.08.2021**
(21) Application number: 17709030.5
(22) Date of filing: 02.03.2017
(51) Int. Cl.: B65B 55/02, A61L 2/26, B65G 17/46, A61L 2/20, A61L 2/07

(54) **TRANSPORT GROUP FOR CONTAINER STERILIZATION APPARATUSES**
TRANSPORTGRUPPE FÜR BEHÄLTERSTERILISATIONSVORRICHTUNGEN
GROUPE DE TRANSPORT POUR APPAREILS DE STÉRILISATION DE RÉCIPIENT

(30) Priority: 03.03.2016 IT UA20161312 U
(43) Date of publication of application: 09.01.2019
(73) Proprietor: I.M.A. Industria Macchine Automatiche S.p.A., 40064 Ozzano dell'Emilia (BO) (IT)
(72) Inventor: MEONI, Eddi, 40064 Ozzano Dell'Emilia (IT)
(74) Representative: Paglia, Pietro
(86) International application number: PCT/EP2017/054943
(87) International publication number: WO 2017/149088

(56) References cited:
- WO-A1-03/051759
- WO-A1-2011/072628
- DE-A1- 3 701 079
- FR-A1- 2 857 350
- JP-A- 2012 200 376
- JP-U- S54 127 377
- US-A- 5 337 886

## Description

The present invention relates to a transport group for container sterilization apparatuses.

Sterilization apparatuses of the known type generally comprise a feed station (through which the containers are introduced on an internal conveyor), a sterilization station (which corresponds to a superheating of an internal chamber in which the containers are located), and a cooling station (which is necessary in order to reduce the temperature of the containers within a range that does not damage the products that said containers are designed to contain).

The containers can be bottles, test tubes, vials, syringes, carpules, jars and the like, and therefore can have different shapes and dimensions: the sterilization apparatus must be able to convey them all, i.e., it must be versatile enough to process all the types of container in all the corresponding possible sizes. WO 03/051759 A1 discloses a transport group for containers filled with liquid foods comprising the technical features of the preamble of claim 1.

Internal conveyors are currently of the format-dependent type, i.e., must be replaced when the shape and size of the container to be sterilized changes.

If the conveyor is not changed when the shape and size of the container changes, problems in the stability of the container can arise and, in the case of conveyors suitable to transport the bulkiest containers, these conveyors are scarcely utilized if it is necessary to convey small containers. A partition is in fact provided on the conveyor between each container and the adjacent one and corresponds to the size of the bulkiest container, with the consequence of having many empty spaces on said conveyor during the conveyance of small containers.

Any constructive solutions that allow to utilize in the best possible manner the conveyance surface of the conveyor entail complex operations for changing format when passing from one type of container to another: it is therefore observed that in any case even these constructive architectures are not versatile and simple to utilize.

The aim of the present invention is to solve the problems described above, by proposing a transport group for container sterilization apparatuses that is suitable to convey containers having substantially any shape and size.

Within this aim, an object of the invention is to propose a transport group for container sterilization apparatuses that allows to maximize the number of containers conveyed simultaneously as a function of their dimensions.

Another object of the invention is to propose a transport group for container sterilization apparatuses that does not require complex operations for changing format.

Another object of the invention is to propose a transport group for container sterilization apparatuses that is easy to use.

A further object of the present invention is to provide a transport group for container sterilization apparatuses that has relatively modest costs, is relatively simple to provide in practice and is safe in application.

This aim and these and other objects which will become better apparent hereinafter are achieved by a transport group for container sterilization apparatuses according to claim 1.

Preferred embodiments of the invention are defined in the dependent claims.

Further characteristics and advantages of the invention will become better apparent from the description of some preferred but not exclusive embodiments of the transport group for container sterilization apparatuses according to the invention, illustrated by way of nonlimiting example in the accompanying drawings, wherein:
Figure 1 is a perspective view of a transport group for container sterilization apparatuses according to the invention, with some parts removed for the sake of clarity;
Figure 2 is an enlarged-scale view of a detail of Figure 1;
Figure 3 is a perspective view of a supporting strip of the group of Figure 1;
Figure 4 is a side view of the strip of Figure 3;
Figure 5 is a sectional side view, taken along a transverse plane, of the strip of Figure 3;
Figure 6 is a perspective top view of a constructive variation of a transport group for container sterilization apparatuses according to the invention;
Figure 7 is a perspective view, taken from below, of the constructive variation of Figure 6.

With reference to the figures, a transport group 1 for apparatuses for sterilizing containers A comprises a conveyor belt 2, which forms an operational plane and advances along an advancement direction, and a plurality of support elements 3, which are mutually separated and each of which comprises a plurality of shaped seats 4 for housing temporarily respective containers A.

Each support element 3 is integral with the respective portion of the conveyor belt 2 on the operational plane and is separated by adjacent support elements 3.

The support elements 3 advance integrally with the conveyor belt 2 along the advancement direction.

The support elements 3 have a transverse longitudinal development that is advantageously perpendicular with respect to the advancement direction and lies on a plane that is parallel to the operational plane of the conveyor belt 2. The shaped seats 4 are arranged on the support element 3 along a direction that is parallel to said longitudinal extension.

The shaped seats 4 have a substantially V-shaped cross-section, such as to be able to accommodate containers of different size within a predetermined range.

The shaped seats 4 further have a longitudinal extension that is inclined with respect to the advancement direction, so that the containers A are inclined with respect to said advancement direction when they are moved by the conveyor belt 2.

Advantageously, the shaped seats 4 have a longitudinal extension that is inclined with respect to the advancement direction and lies on a plane that is perpendicular to the operational plane and is parallel to the advancement direction.

The containers A make contact with the respective shaped seats 4 in at least two points, advantageously along two lines.

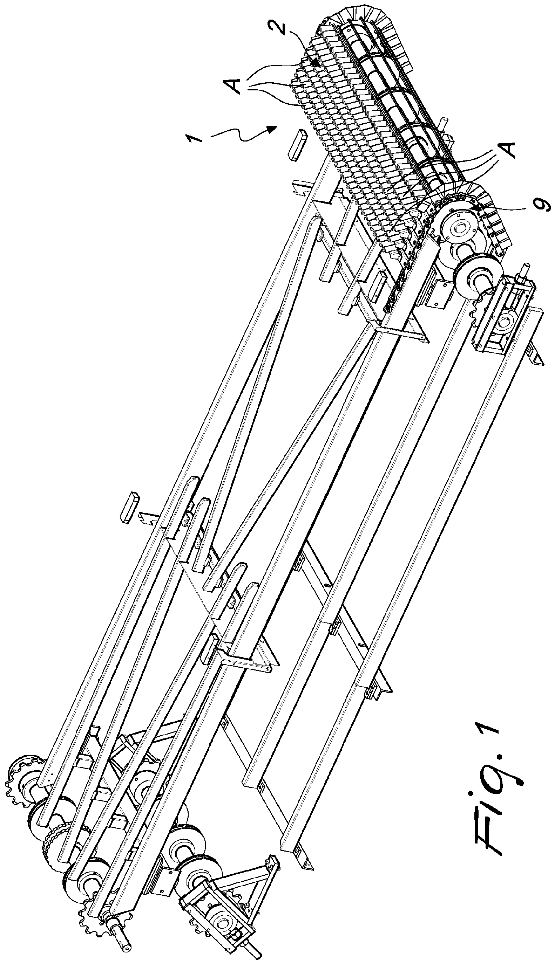

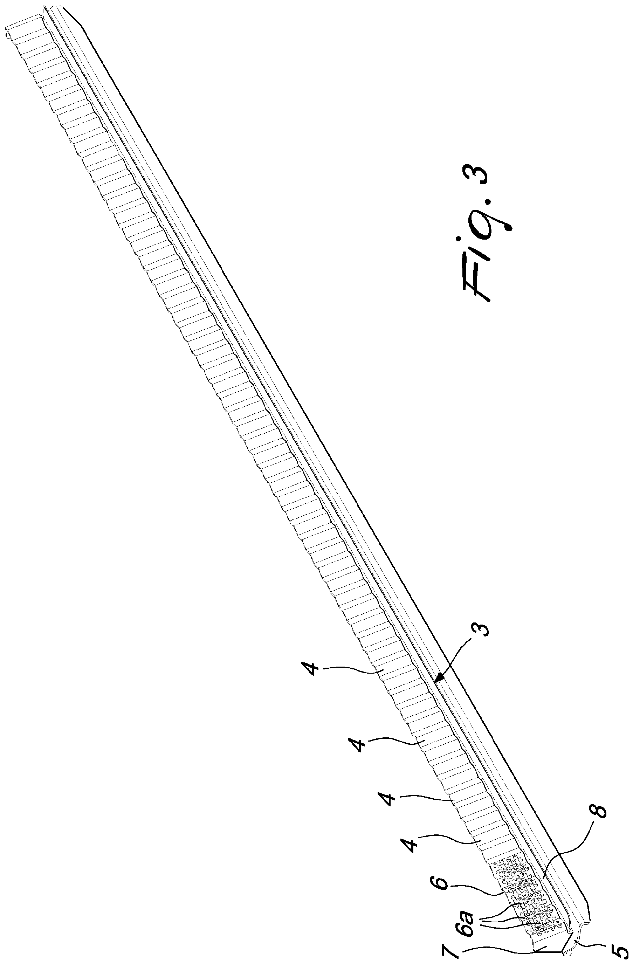

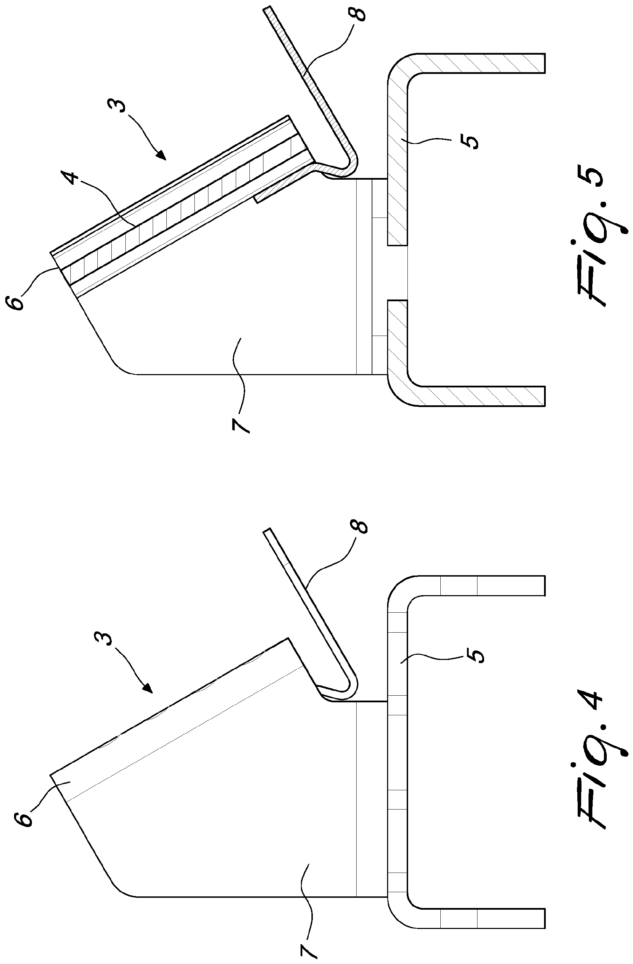

According to a particular constructive solution of unquestionable interest in practice and in application, shown in Figures 1 to 5, each support element 3 can validly comprise a base laminar element 5, which is integral with the conveyor belt 2, and a shaped plate 6 that is integral with the base laminar element 5. Advantageously, the shaped plate 6 protrudes and is substantially inclined with respect to the base laminar element 5. The shaped plate 6 can advantageously be made of metal plate (or other material suitable to provide sheets and plates of low thickness).

Advantageously, at least one portion of the shaped plate 6 can validly be provided with through holes 6a and/or with through slots in order to reduce heat inertia.

Advantageously, the shaped plate 6 comprises a series of concave portions which are alternated with a corresponding series of convex portions, which form a series of contiguous recesses arranged along a direction that is transverse, advantageously perpendicular, with respect to the advancement direction of the conveyor belt 2 and is parallel to the operational plane. In other words, the shaped plate 6 comprises a series of contiguous folds which form respective recesses. Substantially, the shaped plate 6 comprises a succession of consecutive tracts which are mutually inclined so as to form a continuous succession of concavities, which constitutes the shaped seats 4, and convexities.

This configuration is very simple to provide, even by using stainless steel or other alloys suitable to tolerate being subjected to onerous thermal cycles (continuous entry and exit in and from the sterilization chamber) and being subjected to aggressive atmospheres (for example the presence of hydrogen peroxide vapors).

The possibility to provide the support elements 3 using a nonmetallic element material, such as for example a polymeric material and/or a composite material, is not excluded.

Each recess can constitute the shaped seat 4. Each shaped seat 4 is suitable to accommodate temporarily a respective container A.

Each recess is formed between two converging planar portions of the shaped plate 6, which have a substantially V-shaped cross-section, such as to be able to accommodate containers of different size within a predetermined range.

This constructive solution is particularly interesting since it allows to convey easily containers A having the most disparate shapes and dimensions. In particular, the containers are inclined with respect to a vertical direction and are supported in a lower region and laterally by the support element 3.

In the case of relatively small containers A, they are distributed so as to occupy each shaped seat 4 of the shaped plate 6; containers A of relatively large size are distributed so as to leave a predefined number of empty seats 4 between adjacent containers A.

It is deemed useful to point out that between the base laminar element 5 and the shaped plate 6 there are interposed support ribs 7 which are substantially perpendicular to the base laminar element 5.

These support ribs 7 ensure that no flexing of the shaped plate 6 occurs when a large number of containers A is loaded onto it.

The support elements 3 further comprise a lower ledge 8, which is advantageously perpendicular to the shaped plate 6.

Each container A is housed in a respective shaped seat 4, resting in a lower region on the ledge 8 with one of its bases and resting laterally against the shaped seat 4 with at least one of its lateral portions.

Advantageously, each support element 3 is coupled to at least one driving element 9, of the type preferably selected from a chain, a belt, a cable and the like, operatively associated with a drive unit.

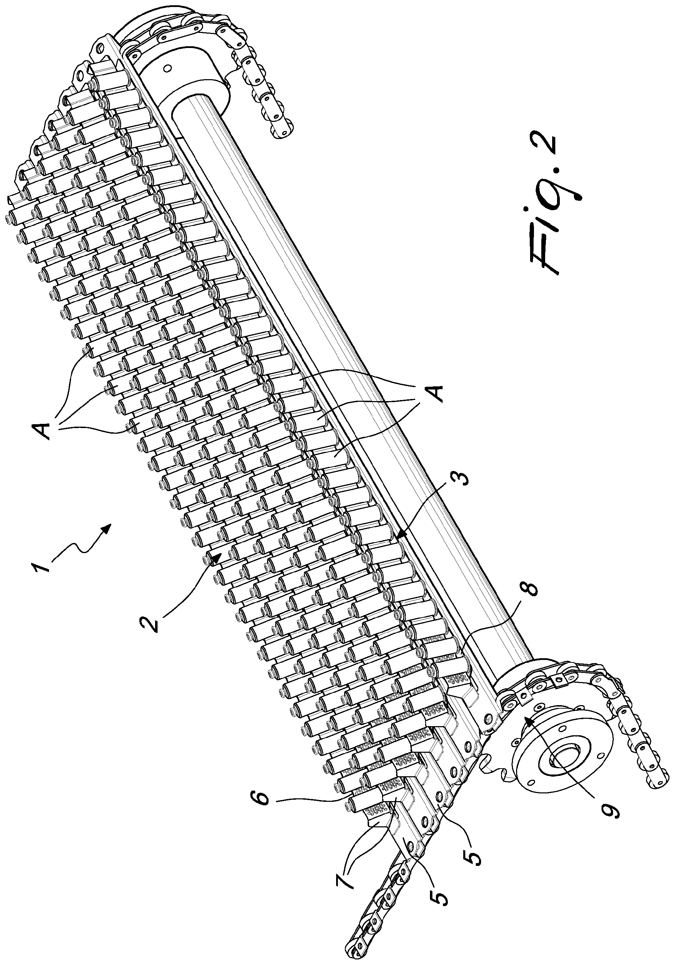

It is sufficient for the ends of each support element 3 to be associated with the driving element 9 (hypothetically a chain, as shown by way of nonlimiting example in Figures 1 and 2) in order to ensure that the support elements 3 can advance in mutually parallel conditions of mutual parallel arrangement and can slide inside, for example, a sterilization chamber.

The transport group 1 is therefore suitable to slide within a sterilization chamber, so that the containers A supported by the support elements 3 undergo a sterilization and/or depyrogenation treatment.

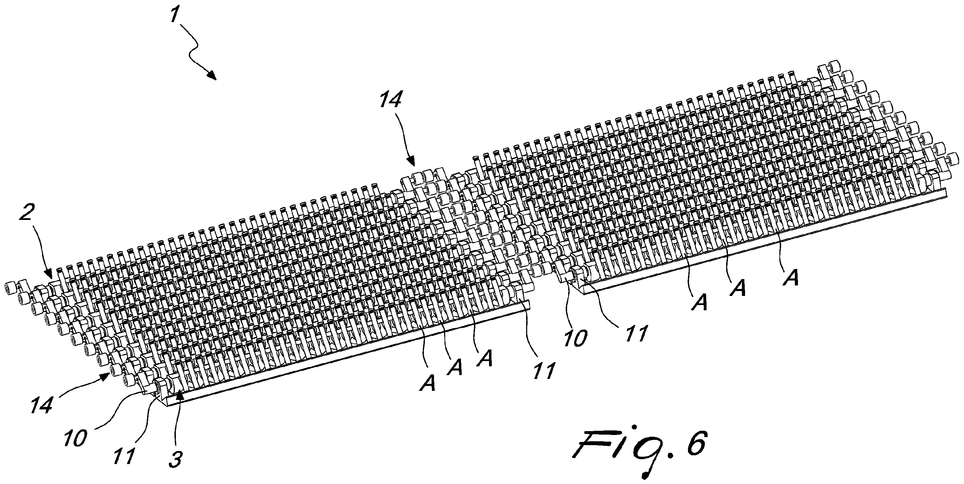

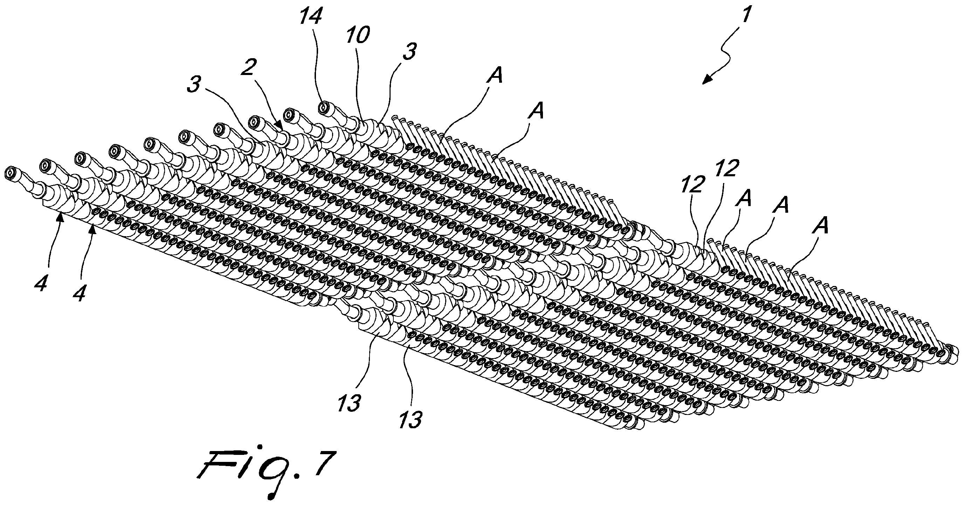

According to an alternative constructive solution, shown in Figures 6 and 7, each support element 3 can advantageously comprise a bar 10, which has a shape that is selected preferably from prismatic, cylindrical, frusto-conical and the like.

The bars 10 can move by rotating about their own longitudinal axis.

The bars 10 are coupled rotatably, by means of hinges 11, to the conveyor belt 2.

Each bar 10 is provided with a peripheral surface on which notches are formed which define the shaped seats 4. Preferably, the notches have converging walls with a substantially V-shaped profile, such as to be able to accommodate containers of a different size within a predetermined range. The notches are aligned along at least one generating line.

Advantageously, each bar 10 comprises at least one plurality of first notches 12 and a plurality of second notches 13, which are arranged on respective and mutually different generating lines.

The first notches 12 are different from the second notches 13; in particular, the first notches 12 can differ from the second notches 13 in terms of size and/or shape.

For example, the first notches 12 have a depth (and therefore a width) that is smaller than that of the second notches 13: the first notches 12 are therefore suitable to house containers A that are smaller than the containers A that can be housed in the second notches 13. Obviously, due to their size difference, the first notches 12 are arranged on the bar 10 according to a spacing that is different from, in particular smaller than, the spacing along which the second notches 13 are arranged.

According to the constructive solution that has just been defined, the bars 10 aligned and coupled rotatably to the conveyor belt 2 are a plurality, all provided with first notches 12 and second notches 13, respectively having the same shape and dimensions, which are aligned and laterally adjacent along respective generating lines.

Each bar 10 comprises a respective orientation element 14 in order to rotate, as a consequence of a specific external action, and arrange in the operational position the notches (the first ones 12 or the second ones 13) that are suitable to house the containers A.

With respect to the preceding constructive solution, shown in Figures 1-5, the present alternative constructive solution has the additional advantage of being able to vary the inclination of the containers A by means of the orientation elements 14, which can vary in rotation the operational position of the notches 12, 13.

It is evident that small containers A can be housed equally in the first notches 12 or in the second notches 13, but due to the different spacing according to which the first and second notches 12 and 13 are arranged, it is advantageous, in order to maximize the number of conveyed containers A, to use the first notches 12 as long as this is allowed by the size of the containers A.

The orientation element 14 can be constituted by an arm that protrudes in a radial direction from the respective bar 10: the end of the arm comprises validly a probe, or a roller, which moves along a cam. All the bars 10 are provided with respective arms provided with corresponding probes and are mutually aligned. The path of the cam, on which the probes of all the bars 10 act, therefore determines the rotation of each bar 10 and the orientation of the provided notch 12 or 13.

The present invention extends its protection also to a sterilization apparatus for containers A which comprises a feed station, a sterilization station, a cooling station, and a group 1 for the transport of containers A as described above, which passes through the stations.

Advantageously, the present invention solves the problems described previously, proposing a transport group 1 for apparatuses for sterilizing containers A that is suitable to convey containers A of substantially any shape and size.

The shaped seats 4 formed in the group 1 according to the invention in fact can accommodate containers A of a shape and size that can vary within a relatively broad range.

In particular, the first constructive embodiment described, which comprises the shaped plate 6, comprises a plurality of contiguous shaped seats 4 within which it is possible to house (with a density that is inversely proportional to the diameter) containers of various shapes and sizes. For example, it is possible to accommodate relatively small containers (for example vials) mutually side by side, arranging one in each shaped seat 4 formed on each shaped plate 6; for relatively large containers (for example bottles) it is instead necessary to leave a certain number of empty shaped seats 4 (at least one) between adjacent containers.

In any case, advantageously, the bases of the containers A (be they vials, bottles, carpules, syringes, jars and the like) rest on the lower ledge 8.

In the case of the second embodiment described, which comprises the bars 10, it is instead possible to house small containers A by rotating the bars 10 (by means of the orientation element 14) so that the seats 12 are directed laterally in the operational position, or it is possible to house large containers A by rotating the bars 10 (by means of the orientation element 14) so that the seats 13 are directed laterally in the operational position.

Advantageously, therefore, the transport group 1 according to the invention allows to maximize the number of containers that are conveyed simultaneously as a function of their dimensions. It has in fact been shown that the surface density of containers A provided by the transport group 1 depends uniquely on the dimensions of the containers A.

In particular, by means of the group 1 according to the invention it is always possible to approximate the maximum surface density of containers A, arranging them so that they are at the minimum possible mutual distance while avoiding mutual contact.

The particular structural shape and the characteristics of the transport group 1 according to the invention ensure that no complex operations are required to change format.

In the provided embodiment of the shaped plate 6 no operation is necessary upon the change of format of the container A to be transported.

Conveniently, the transport group 1 according to the invention is easy to use.

Finally, validly, the transport group 1 according to the invention is relatively simple to provide in practice, bearing substantially modest costs: these characteristics make it an innovation of assured application.

By virtue of the particular shape of the transport group 1 according to the invention, it is appropriate to specify that the movement of the containers A (in particular their arrangement in the seats 4 of the element 3 and their respective pickup) can be performed positively by means of a handling robot (for example an anthropomorphic robot): this possibility is ensured by the fact that the containers A are simply rested in their respective seats 4 (they are not engaged, since the seat 4 does not form a housing that is shaped complementarily to the individual container A but it forms a region at which it can be simply rested stably).

The invention thus conceived is susceptible of numerous modifications and variations within the scope of the appended claims.

In the exemplary embodiments shown, individual characteristics, given in relation to specific examples, may actually be interchanged with other different characteristics that exist in other exemplary embodiments.

In practice, the materials used, as well as the dimensions, may be any according to the requirements and the state of the art.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A transport group for container (A) sterilization apparatuses comprising:
- a conveyor belt (2), which defines an operational plane and advances along an advancement direction;
- a plurality of support elements (3), mutually separated, and integral and movable with the conveyor belt (2), each support element (3) having longitudinal development, transverse to the advancement direction and lying along a direction parallel to the operational plane,
**characterized in that** each support element (3) defines a plurality of shaped seats (4) arranged along said longitudinal development, each shaped seat (4) being adapted to temporarily house a respective container (A), said shaped seats (4) having a substantially "V" shaped cross section, such as to house containers (A) of different size within a predetermined range, said shaped seats (4) having a longitudinal development inclined with respect to the advancement direction, so that the containers (A), temporarily housed in respective said shaped seats (4), result inclined with respect to said advancement direction when they are moved by said conveyor belt (2).

2. The transport group according to claim 1, **characterized in that** said shaped seats (4) comprise at least two contact points, and preferably two contact lines, for contacting the respective containers (A).

3. The transport group according to any one of the preceding claims, **characterized in that** each said support element (3) is coupled to at least one driving element (9), of the type preferably selected from a chain, a belt, a cable and the like, operatively associated with a drive unit.

4. The transport group according to any one of the preceding claims, **characterized in that** each said support element (3) comprises a base laminar element (5), integral with said conveyor belt (2), and a shaped plate (6), protruding and substantially inclined with respect to said base laminar element (5); said shaped plate (6) comprising a succession of mutually inclined consecutive tracts which realize a continuous succession of concavities, defining said shaped seats (4), and convexities.

5. The transport group according to claim 4, **characterized in that** between said base laminar element (5) and said shaped plate (6) are interposed support ribs (7) substantially orthogonal to said base laminar element (5).

6. The transport group according to any one of claims 1-3, **characterized in that** each said support element (3) comprises a bar (10), preferably with a shape selected between prismatic, cylindrical, frusto-conical and the like, rotatably bound, by means of hinges (11), to said conveyor belt (2), along a peripheral surface of each said bar (10) being defined notches (12, 13) which define said shaped seats (4).

7. The transport group according to claim 6, **characterized in that** each said bar (10) comprises a plurality of said first notches (12) and a plurality of said second notches (13), arranged along respective generating lines, mutually different, said first notches (12) being different from said second notches (13).

8. The transport group according to any one of claims 6 and 7, **characterized in that** each said bar (10) comprises an orientation element (14), in order to rotate, as a result of a specific external action, and arrange in operative position said notches (12, 13) suitable for housing the containers (A).

9. A sterilization apparatus for containers (A) comprising a feeding station for feeding said containers (A), a sterilization station for sterilizing said containers (A), a cooling station for cooling said containers (A) and a transport group (1) of said containers (A) according to any one of the preceding claims, said transport group (1) passing through said feeding station, said sterilization station and said cooling station.

## Patentansprüche

1. Transportgruppe für Behälter-(A)-Sterilisationsvorrichtungen, umfassend:
- ein Förderband (2), das eine Arbeitsebene definiert und sich entlang einer Vorschubrichtung fortbewegt;
- eine Vielzahl von Stützelementen (3), die voneinander getrennt und einstückig und mit dem Förderband (2) bewegbar sind, wobei jedes Stützelement (3) eine Längserstreckung aufweist, die quer zur Vorschubrichtung ist und entlang einer Richtung parallel zur Arbeitsebene liegt,
**dadurch gekennzeichnet, dass** jedes Stützelement (3) eine Vielzahl von geformten Sitzen (4) definiert, die entlang der Längserstreckung angeordnet sind, wobei jeder geformte Sitz (4) angepasst ist, um vorübergehend einen entsprechenden Behälter (A) aufzunehmen, wobei die geformten Sitze (4) einen im Wesentlichen "V"-förmigen Querschnitt aufweisen, um Behälter (A) unterschiedlicher Größe innerhalb eines vorbestimmten Bereichs aufzunehmen, wobei die geformten Sitze (4) eine bezüglich der Vorschubrichtung geneigte Längserstreckung aufweisen, so dass die Behälter (A), die zeitweilig in den jeweiligen geformten Sitzen (4) untergebracht sind, in Bezug auf die Vorschubrichtung geneigt sind, wenn sie durch das Förderband (2) bewegt werden.

2. Transportgruppe nach Anspruch 1, **dadurch gekennzeichnet, dass** die geformten Sitze (4) mindestens zwei Kontaktstellen und vorzugsweise zwei Kontaktlinien zur Kontaktierung der jeweiligen Behälter (A) aufweisen.

3. Transportgruppe nach einem der vorhergehenden Ansprüche, **dadurch gekennzeichnet, dass** jedes Stützelement (3) mit mindestens einem Antriebselement (9) gekoppelt ist, vorzugsweise ausgewählt aus einer Kette, einem Riemen, einem Seil und dergleichen, die operativ mit einer Antriebseinheit verbunden sind.

4. Transportgruppe nach einem der vorhergehenden Ansprüche, **dadurch gekennzeichnet, dass** jedes Stützelement (3) ein mit dem Förderband (2) integrales Basislamellenelement (5) und eine geformte Platte (6) umfasst, die in Bezug auf das Basislamellenelement (5) hervorsteht und im Wesentlichen geneigt ist; wobei die geformte Platte (6) eine Folge von zueinander geneigten aufeinanderfolgenden Bahnen umfasst, die eine kontinuierliche Folge von Konkavitäten realisieren, die die geformten Sitze (4) und Konvexitäten definieren.

5. Transportgruppe nach Anspruch 4, **dadurch gekennzeichnet, dass** zwischen dem Basislamellenelement (5) und der geformten Platte (6) im Wesentlichen orthogonal zu dem Basislamellenelement (5) Stützrippen (7) eingefügt sind.

6. Transportgruppe nach einem der Ansprüche 1-3, **dadurch gekennzeichnet, dass** jedes der Stützelemente (3) eine Stange (10) umfasst, vorzugsweise mit einer Form ausgewählt zwischen prismatisch, zylindrisch, kegelstumpfförmig und dergleichen, die drehbar mittels Scharnieren (11) an das Förderband (2), wobei entlang einer Umfangsfläche jeder der Stangen (10) definierte Kerben (12, 13) sind, die die geformten Sitze (4) definieren.

7. Transportgruppe nach Anspruch 6, **dadurch gekennzeichnet, dass** jede der Stangen (10) eine Vielzahl der ersten Kerben (12) und eine Vielzahl der zweiten Kerben (13) umfasst, die entlang jeweiliger Erzeugungslinien angeordnet sind, die voneinander verschieden sind, wobei sich die ersten Kerben (12) von den zweiten Kerben (13) unterscheiden.

8. Transportgruppe nach einem der Ansprüche 6 und 7, **dadurch gekennzeichnet, dass** jede Stange (10) ein Orientierungselement (14) umfasst, um sich infolge einer spezifischen äußeren Einwirkung zu drehen und die Kerben (12, 13) in Betriebsstellung anzuordnen, die geeignet sind, die Behälter (A) aufzunehmen.

9. Sterilisationsvorrichtung für Behälter (A) mit einer Zuführstation zum Zuführen der Behälter (A), einer Sterilisationsstation zum Sterilisieren der Behälter (A), einer Kühlstation zum Kühlen der Behälter (A) und einer Transportgruppe (1) der Behälter (A) nach einem der vorhergehenden Ansprüche, wobei die Transportgruppe (1) die Zuführstation, die Sterilisationsstation und die Kühlstation passiert.

## Revendications

1. Groupe de transport pour appareils de stérilisation de récipient (A) comprenant :
- une bande transporteuse (2) qui définit un plan fonctionnel et qui avance le long d'une direction d'avancement ;
- une pluralité d'éléments de support (3), séparés les uns des autres, et formés d'un seul tenant et mobiles avec la bande transporteuse (2), chaque élément de support (3) ayant un développement longitudinal, transversal à la direction d'avancement et se trouvant le long d'une direction parallèle au plan fonctionnel,
**caractérisé en ce que** chaque élément de support (3) définit une pluralité de sièges façonnés (4) agencés le long dudit développement longitudinal, chaque siège façonné (4) étant conçu pour loger provisoirement un récipient respectif (A), lesdits sièges façonnés (4) ayant une section transversale sensiblement en forme de « V », de manière à loger des récipients (A) de tailles différentes dans une plage prédéterminée, lesdits sièges façonnés (4) ayant un développement longitudinal incliné par rapport à la direction d'avancement, de sorte que les récipients (A), temporairement logés dans lesdits sièges façonnés respectifs (4), s'inclinent par rapport à ladite direction d'avancement lorsqu'ils sont déplacés par ladite bande transporteuse (2).

2. Groupe de transport selon la revendication 1, **caractérisé en ce que** lesdits sièges façonnés (4) comprennent au moins deux points de contact, et de préférence deux lignes de contact, pour entrer en contact avec les récipients respectifs (A).

3. Groupe de transport selon l'une quelconque des revendications précédentes, **caractérisé en ce que** chacun desdits éléments de support (3) est couplé à au moins un élément d'entraînement (9), dont le type est choisi de préférence parmi une chaîne, une courroie, un câble et analogue, et lequel élément d'entraînement est fonctionnellement associé à une unité d'entraînement.

4. Groupe de transport selon l'une quelconque des revendications précédentes, **caractérisé en ce que** chacun desdits éléments de support (3) comprend un élément laminaire de base (5), formé d'un seul tenant avec ladite bande transporteuse (2), et une plaque façonnée (6), faisant saillie et étant sensiblement inclinée par rapport audit élément laminaire de base (5) ; ladite plaque façonnée (6) comprenant une succession de segments consécutifs mutuellement inclinés qui réalisent une succession continue de concavités, définissant lesdits sièges façonnés (4), et de convexités.

5. Groupe de transport selon la revendication 4, **caractérisé en ce qu'**entre ledit élément laminaire de base (5) et ladite plaque façonnée (6) sont interposées des nervures de support (7) sensiblement orthogonales par rapport audit élément laminaire de base (5).

6. Groupe de transport selon l'une quelconque des revendications 1 à 3, **caractérisé en ce que** chacun desdits éléments de support (3) comprend une barre (10), de préférence ayant une forme choisie entre prismatique, cylindrique, tronconique et analogue, liée de manière rotative, au moyen de charnières (11), à ladite bande transporteuse (2), le long d'une surface périphérique de chacune desdites barres (10) étant définies des encoches (12, 13) qui définissent lesdits sièges façonnés (4).

7. Groupe de transport selon la revendication 6, **caractérisé en ce que** chacune desdites barres (10) comprend une pluralité desdites premières encoches (12) et une pluralité desdites secondes encoches (13), agencées le long de lignes de génération respectives, mutuellement différentes, lesdites premières encoches (12) étant différentes desdites secondes encoches (13).

8. Groupe de transport selon l'une quelconque des revendications 6 et 7, **caractérisé en ce que** chacune dites barres (10) comprend un élément d'orientation (14), afin de tourner, à la suite d'une action extérieure spécifique, et d'agencer en position de fonctionnement lesdites encoches (12, 13) aptes à loger les récipients (A) .

9. Appareil de stérilisation de récipients (A) comprenant une station d'alimentation pour alimenter lesdits récipients (A), une station de stérilisation pour stériliser lesdits récipients (A), une station de refroidissement pour refroidir lesdits récipients (A) et un groupe de transport (1) desdits récipients (A) selon l'une quelconque des revendications précédentes, ledit groupe de transport (1) passant par ladite station d'alimentation, ladite station de stérilisation et ladite station de refroidissement.
